# EUROPEAN PATENT SPECIFICATION

(11) **EP 2 652 124 B1**
(45) Date of publication and mention of the grant of the patent: **29.07.2015**
(21) Application number: 11802902.4
(22) Date of filing: 16.12.2011
(51) Int. Cl.: C12N 5/00, B82Y 5/00

(54) **CELL CARRIER, ASSOCIATED METHODS FOR MAKING CELL CARRIER AND CULTURING CELLS USING THE SAME**
ZELLSUBSTRAT, ZUGEHÖRIGE VERFAHREN ZUR HERSTELLUNG EINES ZELLSUBSTRATS UND KULTIVIERUNG VON ZELLEN DAMIT
SUPPORT CELLULAIRE, PROCÉDÉS ASSOCIÉS POUR FABRIQUER UN SUPPORT CELLULAIRE ET CULTIVER DES CELLULES À L'AIDE DUDIT SUPPORT

(30) Priority: 16.12.2010 US 970735
(43) Date of publication of application: 23.10.2013
(73) Proprietor: General Electric Company, Schenectady, NY 12345 (US)
(72) Inventor: RANGARAJAN, Arvind, Niskayuna, NY 12309 (US); SUSARLA, Prameela, Manvel, TX 77578 (US); MILLER, Scott Michael, Niskayuna, NY 12309 (US); SMITH, Reginald Donovan, Niskayuna, NY 12309 (US); RUBINSZTAJN, Slawomir, Niskaynua, NY 12309 (US); BELETSKII, Anton, Niskayuna, NY 12309 (US)
(74) Representative: Aldenbäck, Ulla Christina
(86) International application number: PCT/EP2011/073064
(87) International publication number: WO 2012/080473

(56) References cited:
- WO-A1-00/70406
- WO-A1-2008/106771
- WO-A2-2004/090506
- WO-A2-2006/033935
- US-A1- 2003 219 824
- SARA LINDSTRÖM ET AL: "High-Density Microwell Chip for Culture and Analysis of Stem Cells", PLOS ONE, vol. 4, no. 9, E6997, 1 January 2009 (2009-01-01), pages 1-9, XP055022128, ISSN: 1932-6203, DOI: 10.1371/journal.pone.0006997
- BD Biosciences: "BD BioCoat - Dish 35mm PLL 5Pac 20cas", Catalog page , 2010, XP002671673, Retrieved from the Internet: URL:http://www.bdbiosciences.com/ptProduct .jsp?prodId=364945&key=dish+35mm+pll&param =search&mterms=true&from=dTable [retrieved on 2012-03-16]

## Description

### FIELD

The invention relates to cell carriers, and associated methods for making and using the cell carriers. More particularly, the invention relates to polymer based cell carriers for cell growth.

### BACKGROUND

Adherent cells have conventionally been grown on glass surfaces or on polymer substrates. Surfaces for cell culture are often pre-treated to enhance cell adhesion and proliferation. A wide variety of static culture vessels is available for adherent cell culture in the laboratory. While static culture vessels such as T-flasks, Cell Factory (Nunc) or Cell Stack® (Corning®) units do allow for some scale-up of adherent cell culture, they become limiting at larger scales as they are labor-intensive, subject to variability due to manual processing, and limited in volumetric productivity (e.g. cell yield per volume of incubator space).

Cell culture using bioreactors has long been practiced as the preferred scale-up method for cell culture. The use of microcarriers for adherent cell culture is common in industrial practice, such as in bioprocessing. Microcarrier beads have been developed to provide increased surface area for cell attachment, and to enable high-density adherent cell culture on an industrial scale. Typical bioreactor vessels employ some means of agitation, such as internal impellers, rocking or shaking mechanisms to suspend the cells and allow mass transfer of nutrients, oxygen and metabolic waste products. However, agitation can subject cells to high degrees of flow-induced stress that can damage cells, especially sensitive ones such as certain mammalian cell lines and primary cells. Flow-induced stresses in bioreactors can arise due to relative motion of the liquid medium with respect to vessel walls, impellers, or other vessel components, and due to relative motion of carriers with respect to the medium. Cells may also be damaged in bioreactor vessels with internal moving parts if microcarriers with cells collide with one another, or with vessel components, or agitator components. Therefore, carriers that protect cells from agitation-induced damage are desired. Certain carrier designs (e.g. macro porous beads, nonwoven fibrous mats) do offer protection for cells; however, cell visualization and cell recovery from such carriers is difficult.

Therefore, there is a need for a carrier for adherent cell growth that protects cells from the effects of agitation or shear stress and yet facilitates cell expansion, visualization and release. Efficient cell expansion is particularly important for high yield industrial scale cell culture processes for adherent cells, including such shear-sensitive cells as Mesenchymal Stromal Cells (MSCs), which are currently expanded in static culture vessels. Therefore, the development of cell culture carriers that facilitate cell attachment, proliferation and release, and that reduce shear forces on cells is highly desired.

### BRIEF DESCRIPTION

The invention relates to carriers for cell culture and methods of making and using the carriers. One or more embodiments of the carrier for cell culture comprise one or more indentations on two outer surfaces.

One embodiment of a carrier for growing adherent cells, comprises one or more outer surfaces; and one or more structured indentations on the two outer surfaces, wherein the carrier has a length at least about 0.2 mm, a width at least about 0.2 mm, and a height in a range from about 0.05 mm to 1.2 mm and each of the structured indentations has a major axis in a range from about 0.1 mm to 0.5 mm, minor axis in a range from about 0.1 mm to 0.5 mm and depth in a range from about 0.025 mm to about 0.5 mm.

A carrier for growing adherent cells, comprises two outer surfaces; and a single structured indentation on at least one surface, wherein the single structured indentation has a length of at least about 1 mm, a width of at least about 1mm, a height in a range from about 1mm to 10mm, and a wall-thickness of the carrier in a range from about 0.05 mm to 2 mm.

A method of culturing adherent cells, comprises providing a carrier comprising two outer surfaces; and one or more structured indentations on the two outer surfaces, wherein the carrier has a length at least about 0.2 mm, a width at least about 0.2 mm, and a height in a range from about 0.05 mm to 1.2mm and each of the structured indentations has a major axis in a range from about 0.1 mm to 0.5 mm, minor axis in a range from about 0.1 mm to 0.5 mm and depth in a range from about 0.025 mm to about 0.5 mm, and growing the cells on the carrier.

An example of a method of making a carrier for growing cells, comprises a) providing two flat polymer films, b) forming on the two flat polymer films a plurality of structured indentations on at least one surface of each of the two films, c) laminating the two polymer films together (with flat surfaces joined to each other) to form a laminated polymer film with two outwardly facing surfaces comprising a plurality of the structured indentations, d) cutting the laminated polymer film into a plurality of portions, and e) imparting a treatment to the portions comprising one or more of corona discharge treatment, gas plasma treatment, chemical functionalization or coating.

### DRAWINGS

These and other features, aspects, and advantages of the present invention will become better understood when the following detailed description is read with reference to the accompanying drawings in which like characters represent like parts throughout the drawings, wherein:
FIG. 1A is an image of a carrier of the invention comprising a plurality of indentations showing dimensions of the carrier. FIG. 1B is an image of the same carrier showing dimensions of each indentation.
FIG. 2A is an image of a carrier of the invention comprising one indentation on one side of the base. FIG. 2B is an image of a carrier of the invention comprising one indentation each on two opposing sides of the base. FIG. 2C is a scanning electron microscope (SEM) image of a carrier of the invention comprising a plurality of indentations on one side of the base. FIG. 2D is an SEM image of a carrier of the invention comprising a plurality of indentations on both sides of the base.
FIG. 3 is a process flow diagram of an example of methods of making carriers of the invention on a small scale in batch mode.
FIG. 4A is an SEM image of a carrier of the invention with roughly rectangular wall cross-section, and FIG. 4B is an image of a carrier of the invention having an alternate wall shape (roughly triangular wall cross-section).
FIG. 5A is a 100x fluorescence microscopy image of hMSC grown on the carrier of the invention illustrating cell growth on top and bottom of the carrier surfaces at day 1, day 4, day 7, and day 9. FIG. 5B is a graph showing growth of hMSCs on the carriers of the invention in spinner flasks or STRs and comparison to their growth in TCPS under static conditions.
FIG. 6 is a graph illustrating the growth of hMSCs procured from various sources on carriers of the invention in spinner flasks and on tissue culture treated plate surface (TCPS) in static condition as a comparison.
FIG. 7 is a graph illustrating larger scale culture of hMSC in a spinner flask.
FIG. 8A is a series of 100x optical microscopy images of MDCK cells grown on the carrier of the invention, illustrating cell growth on top and bottom of the indentations of the carrier at 24 hours, 96 hours, and 168 hours. FIG. 8B is a graph representing the luminescence signal for cultured MDCK cells grown on the carriers of the invention and on flat carriers as a function of time.
FIG. 9A is a series of 100x optical microscopy images of MRC-5 cells grown on the carrier of the invention illustrating cell growth on top and bottom of the indentations of the carrier at 24 hours, 96 hours, and 168 hours. FIG. 9B is a graph representing the luminescence of cultured MRC-5 cells grown on the carriers of the invention and on flat carriers as a function of time.
FIG. 10A is a series of images of CHO cells grown on the carriers of the invention imaged after 1 day, 4 days, 8 days, and post-trypsin treatment. FIG. 10B is a graph showing CHO cells grown on the carriers of the invention under static conditions, and in a spinner flask.
FIG. 11A is a fluorescence microscope image of hMSCs cultured on the carriers of the invention in spinner flasks, after recovery and adipogenesis. FIG. 11B is a fluorescence microscope image of hMSCs used as control.
FIG. 12 is a graph showing calcium content of hMSCs after osteogenesis in comparison to control cells.

### DETAILED DESCRIPTION

One or more of the embodiments of the invention relate to a carrier for growing adherent cells, wherein the carrier is suspended in a bioreactor wherein the carrier is useful for efficient cell adhesion, cell growth, and cell release. High yield of cells is required in various applications involving cell culture, and this carrier may meet that requirement.

To more clearly and concisely describe the subject matter of the claimed invention, the following definitions are provided for specific terms, which are used in the following description and the appended claims. Throughout the specification, exemplification of specific terms should be considered as non-limiting examples.

The singular forms "a", "an" and "the" include plural referents unless the context clearly dictates otherwise. Approximating language, as used herein throughout the specification and claims, may be applied to modify any quantitative representation that could permissibly vary without resulting in a change in the basic function to which it is related. Accordingly, a value modified by a term such as "about" is not to be limited to the precise value specified. In some instances, the approximating language may correspond to the precision of an instrument for measuring the value. Where necessary, ranges have been supplied, and those ranges are inclusive of all sub-ranges there between.

A "carrier" or "carrier for growing cells", as referred to herein, is a support for adhering and culturing cells. The carrier may have indentations on it. Suitable materials of the carrier may include, but are not limited to, polymers, copolymers or blends of polymers. The carrier may further be coated with a suitable coating material for effective cell adherence and proliferation.

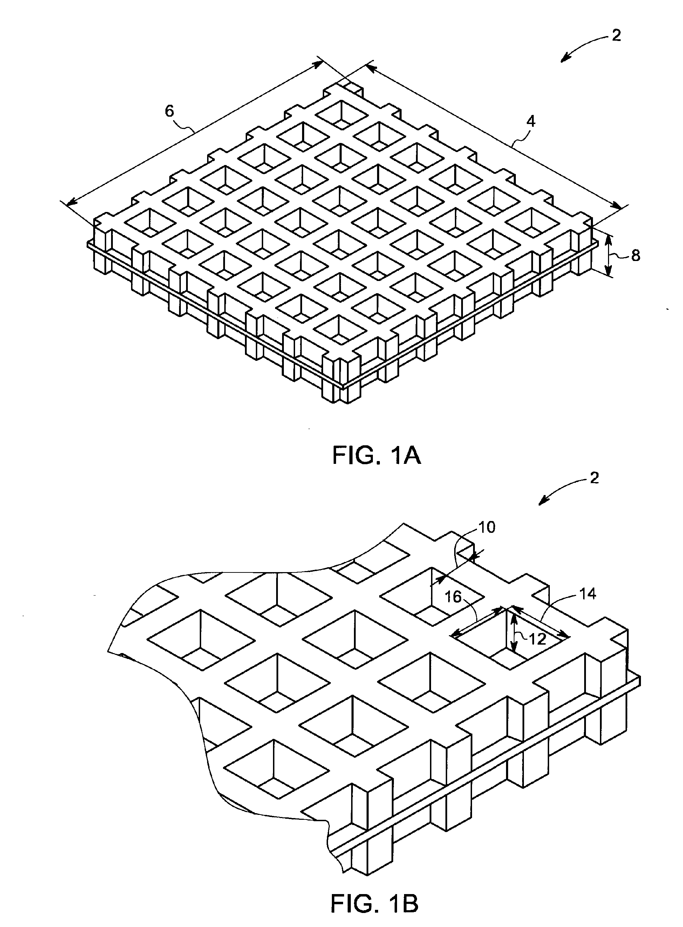

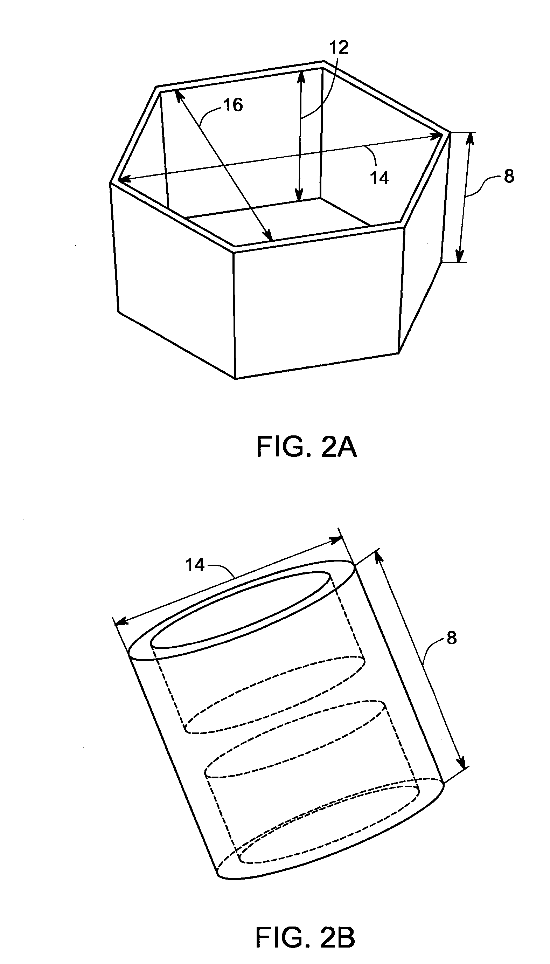

A "major axis", as referred to herein, is the longest dimension of each indentation present on the carrier surface. For example, for a rectangular indentation, length of the indentation is referred as the 'major axis'. A "minor axis", as referred to herein, refers to a dimension other than the longest dimension, of each indentation present on the carrier surface. For example, for a rectangular indentation, width of the indentation is referred as the 'minor axis'. For example, the major axis is same for a square indentation as the length and width are same, as shown in FIG. 1B, 14 and 16 respectively, the major axis is a diameter for a circular indentation as shown in FIG. 2B, 14, major axis is length for a rectangular indentation, and major axis is the major axis of an elliptical indentation.

An "aspect ratio", as referred to herein, is a ratio of depth to major axis of each structured indentation. For example, an aspect ratio for a circular indentation is a ratio of depth to diameter.

Embodiments of the carrier in suspension comprise two outer surfaces; wherein the two outer surfaces of the carrier comprise one or more structured indentations. The invention also comprises methods of making the carrier, and methods and kits for culturing cells using the carriers for cell growth.

The carrier for growing adherent cells, comprises two outer surfaces; and one or more structured indentations in the two outer surfaces, wherein the carrier 2, as shown in FIG. 1A, has a length 4 at least about 0.2 mm, a width 6 at least about 0.2 mm, and a height 8 in a range from about 0.05 mm to 1.2 mm. In some embodiments, the carrier has a length 4 in a range from about 0.2 mm to 5 mm, a width 6 in a range from about 0.2 mm to 5mm, and a height 8 in a range from about 0.05 mm to 1.2 mm. In some embodiments, the carrier has a width and length from about 0.2 to 25 mm. In some embodiments, the wall-thickness 10 of the carrier is in a range from about 0.05 mm to 2 mm.

Embodiments of the structured indentations, as shown in FIG. 1B, comprise a depth 12, a major axis 14, and a minor axis 16, wherein the major axis 14 of an indentation is in a range from about 0.1 mm to 0.5 mm, the minor axis 16 is in a range from about 0.1 mm to 0.5 mm, and the depth 12 is in a range from about 0.025 mm to about 0.5 mm. The wall-thickness 10 is in a range from about 0.05 mm to 2 mm. As used herein the term, 'depth' of an indentation refers to the depth of the inner wall of each indentation. As used herein, the term 'wall-thickness' refers to a thickness of a walls for the carrier with a plurality of the structured indentations as shown in FIG. 1B. Each of the structured indentation has an aspect ratio in a range from about 0.1 to about 1.5.

In one embodiment, the carrier may comprise one indentation on at least one surface of the carrier as shown in FIG. 2A. In this embodiment, the carrier is a 'cup' like structure on one outer surface of the base with a continuous wall surrounding the base of the carrier. In an alternate embodiment, the carrier may comprise one indentation on each of the surfaces of the carrier as shown in FIG. 2B. In this embodiment, the carrier has two 'cup' like structures on opposing outer surfaces of the base with a continuous wall surrounding the cups. This carrier may be useful for specific cell culture conditions or for specific cell-types. The single carrier (FIG 2A and 2B) has a length in a range from about 0.1 mm to 5 mm, a width in a range from about 0.1 to 5 mm, and a height 8 in a range from about 1mm to 10mm, and a wall-thickness 10 of the carrier in a range from about 0.05 mm to 2 mm. In case of a single 'cup' (FIG. 2A) or two 'cups' on opposing sides of the base (FIG. 2B), has a length that is same as the major axis 14 as shown in FIG. 2A and 2B, a width that is same as the minor axis 16, and the cup has a depth 12, as shown in FIG. 2A.

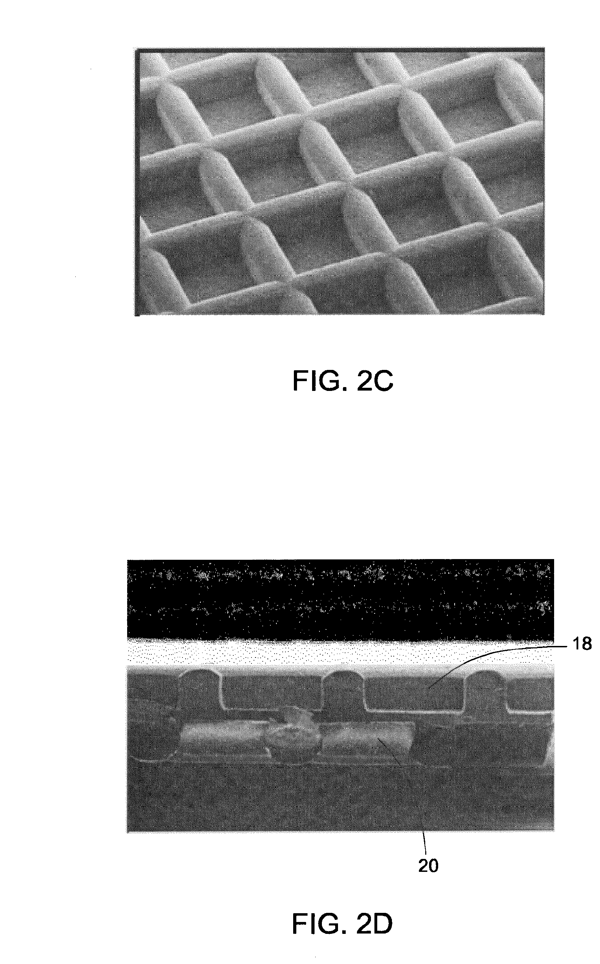

In some embodiments, the carrier comprises at least one surface for growing adherent cells, wherein more than one structured indentation is present on the surface, for example, the carrier has a plurality of structured indentations on one outer surface of the base, as shown an SEM image in FIG. 2C. The carrier, in one embodiment, comprises at least two outer surfaces. In this embodiment, more than one structured indentation is formed on each of the outer surfaces, such as 18 and 20 are the structured indentations on the upper and lower surface respectively, as shown in FIG. 2D. In this embodiment, the carrier has a plurality of indentations on opposing outer surfaces of the base (FIG 2D).

In some embodiments, the carrier has a substantially planar disc-like structure. As used herein, 'substantially planar disc', refers to a disc, which provides a planar surface area for growing cells. The shape of the carrier may be polygonal. In one or more embodiments, the shape of the carrier may vary, for example, the carrier may have an overall perimeter that is circular, elliptical, triangular, rectangular, square, pentagonal, or hexagonal shape.

The disc like-structure of the carrier may provide higher surface area per unit volume for culturing cells, relative to, e.g. spherical structures. The carrier size (both length, and width of 0.2 to 5 mm) may allow about 10 - 100X fold of hMSC expansion per passage. Efficient separation of released (e.g. trypsinized) cells from the carriers is facilitated due to the significant size difference between the cells (-15 micron) and the carriers (larger than 0.2 mm). Released cells may be separated from the carriers via simple filtration, or separation of the supernatant after allowing the carriers to settle.

The structured indentation has a wall that protrudes normal to the outer surface of the carrier, as shown in FIGs. 1A, 1B, 2A, and 2B. The wall height is chosen to balance the various requirements of the carrier, for example, a lower wall (i.e. shallow indentation) allows higher packing density of carriers per unit volume, and therefore can provide higher cell yield per unit volume of reactor. Moreover, transport of oxygen, nutrients and metabolic waste to/from the cells is facilitated at lower wall height (i.e. shallower indentations). However, a higher wall (i.e. deeper indentation) can offer higher degrees of protection from hydrodynamic forces arising due to agitation inside the bioreactor. Further, a higher wall or deeper indentation can provide a microenvironment that prevents dilution of any cell-secreted molecules. This may be advantageous if cell-cell signaling or autocrine factors are a desired part of the cell culture or processing operations. The desired range of the height of the wall projected above the plane of the carrier is therefore optimized with these factors in mind, in a range from 0.05 mm to 1.2 mm; in some embodiments from about 0.05 mm to about 0.5mm, or in some embodiments, from about 0.08mm to about 0.2mm.

The carriers are in suspension inside a bioreactor, comprising a fluid having a convective motion that generates sufficient transport of nutrients and oxygen to cells. The cells adhere to the surface of the structured indentations having a flat or curved wall of sufficient height such that the effect of fluid-induced hydrodynamic stress on the cells is minimized. The carrier comprises an optimum depth of indentations, balancing the needs of the cells providing access to nutrients and metabolites, while protecting the cells from exposure to hydrodynamic shear generated by fluid motion.

The structured indentations also form relief features on the carrier surface. The relief feature may be present on one or more surfaces of the carriers, which prevents the carriers from sticking to each other. Carrier sticking or clumping may be issue with certain types of flat or smooth carriers. The relief features on the carrier also serve to prevent the carriers from sticking to the inner walls of the reactor or culture vessel, which facilitates cleaning the reactors / culture vessels between batches of cell culture.

A cross sectional profile of each indentation may have, as non-limiting examples, a polygonal, a circular, or an elliptical shape. Each of the polygonal indentations may have, as non-limiting examples, a triangular, rectangular, square, pentagonal or hexagonal shape. The dimension of the major axis and minor axis of the indentations may be the same or different.

The carrier may be made of glass, polymer, ceramic, metal or a combination thereof In one embodiment, the carrier is made of a polymer or a copolymer or a blend of polymers. The polymers may comprise, but are not limited to synthetic and natural polymers such as, polyester including polyethylene terephthalate (PET), polystyrene, polycarbonate, polyamide, polyurethane, olefin polymer, dextran, silicone, or polyacrylate, or copolymer or blend of polymers thereof. In one specific embodiment, the carrier is made of polystyrene.

The polymer may be transparent, which allows cell observation under an optical microscope. In certain embodiments, the carrier has a substantially planar disc shape, which facilitates cell visualization by preventing lensing effects. Refraction of light can be a hindrance to visualization of cells on spherical carriers of certain refractive index. Cell visualization is useful, for example, for culturing and monitoring cells during vaccine production or stem cell expansion. In some embodiments, the polymer and surface treatment is substantially free of components of animal origin. This is especially beneficial in therapeutic applications, e.g. in the production of cells for cellular therapies. The polymer may be rigid at room temperature / cell culture temperature, non-porous and may have non-swelling properties in water, PBS or growth medium. The rigid, non-swelling, non-porous properties of the polymer can facilitate cell release, for example, when using standard trypsinization protocols.

The polymer-based carrier surfaces are optionally modified with functional groups or coatings to enable better cell attachment and growth. In some embodiments, a surface treatment is imparted to the embossed polymer film comprising one or more of corona discharge treatment, gas plasma treatment, chemical functionalization or coating. A variety of biomolecules may also be used to modify surfaces of the carriers to enhance cell attachment. Non-limiting examples of the biomolecules include collagen, fibronectin, vitronectin and laminin. In one embodiment, the surfaces are modified with recombinant fibronectin to enhance surface cytophilicity for better attachment of the cells. The surface modification may result in a change, for example, in hydrophobicity or hydrophilicity.

In some embodiments, the surfaces are treated with corona discharge to modify one or more surface properties of the carriers. In corona discharge treatment, a current develops from an electrode with a high potential in a neutral gas, such as air. Ionization of the gas generates a layer of plasma around the electrode. The ions generated eventually pass the charge to nearby areas of lower potential, or recombine to form neutral gas molecules. Surfaces of organic films such as polystyrene, polyesters and others may be oxidized when exposed for a short time to the reactive air plasma by corona discharge surface treatment. Corona discharge treatment can increase the oxygen content on the polymer surface and improve the film wettability by water.

The surface modification may be achieved via plasma treatment. In some embodiments, the surface is treated with of plasma to modify the surface properties of the carrier. Plasma treatment is carried out in a plasma reactor, which is a vacuum vessel with a gas at low pressure, typically 10 to 1000 mTorr. When a high frequency electric field is generated in the reactor, a plasma is formed containing reactive species like ions, free radicals and vacuum-UV photons. These species react with the polymer surface and cause a chemical modification with various properties depending on the nature of the gas and the plasma parameters. Gases such as oxygen, ammonia and argon are typically used for modification of the surfaces and adhesion improvement on polymer surfaces. In one embodiment, the polymer surface is modified by oxygen-plasma treatment to increase the cytophilicity of the surface. The surface functionality may also be altered via wet chemical methods such as oxidation treatments using perchloric acid or permanganate or partial hydrolysis using strong acids or bases.

A coating may also be applied on each of the surfaces to modify the surface property of the carriers, e.g. hydrophobicity, hydrophilicity, or wettabilty. One index of hydrophobicity/hydrophilicity is contact angle of a water droplet on the surface. Contact angle can be measured by techniques well-known in the art. The water contact angle for the coated carrier surface may be in a range from about 10°C to about 90°C, or in some embodiments the water contact angle is from 30°C to 70°C. The carrier surface may be modified, for example, to enhance cell release as well as cell attachment. The coating may be made, for example, of a thermoresponsive polymer, pH responsive polymer, or combination thereof. Thermoresponsive polymers may include, but are not limited to, poly(N-isopropylacrylamide) (PNIPAM), poly(di(ethyleneglycol)methylether methacrylate) (PDEGMA). pH responsive polymers may include, but are not limited to, copolymers of acrylic acid, dimethylaminoethylacrylate, and hydroxyethylacrylate. The coating may comprise one or more layers. In some embodiments, where the coating comprises multiple layers, the layers may be homogeneous or heterogeneous. For one example, one layer may be made of thermoresponsive polymer, and another layer may be made of pH responsive polymer. Thermoresponsive or pH responsive polymer coatings on the surface can facilitate easy release of cultured cells from the carrier surface.

An example of a method of making a carrier for growing cells, comprises providing a plurality of flat films and laminating the flat films to form a solid support. The solid support is subjected, to a method such as embossing, casting thermoforming, or injection molding to form structured indentations. In some embodiments, the solid support is embossed to form structured indentations and make an embossed solid support, which is further treated with a plasma to form a plasma treated embossed solid support, followed by cutting or dicing the plasma treated embossed solid support to a plurality of portions or pieces to form a plurality of carriers. In one example, the embossing of the solid support is performed using a mold.

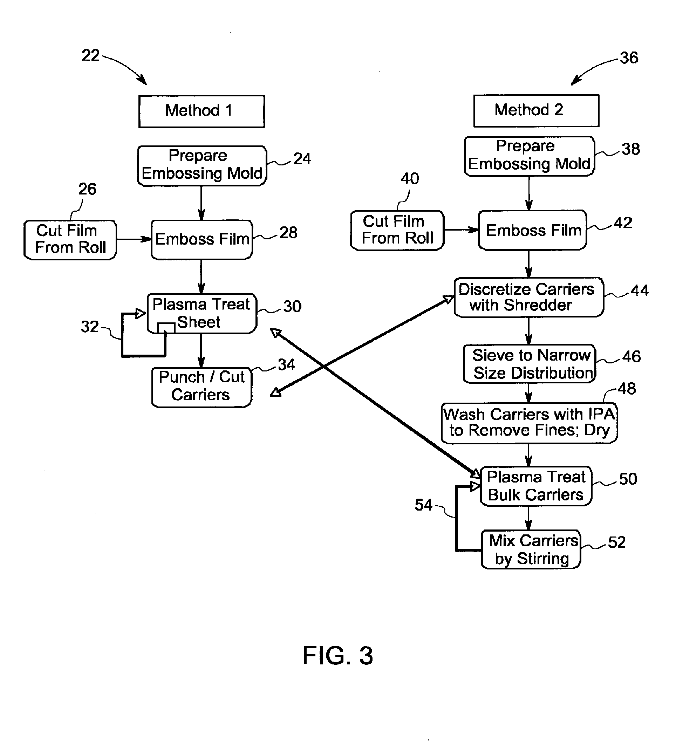

In one example, a process for making a carrier for growing cells is generally illustrated in FIG. 3. The process comprises two alternate methods, method (1) and method (2). The method (1) 22 comprises the steps of preparing embossing mold 24, and cutting a film from a roll 26, followed by embossing the film 28. The embossed film is subjected to a plasma treatment 30 to form a plasma treated embossed film. The embossed film is then optionally plasma treated on the other side of the film for better uniformity of treatment 32. The plasma treated embossed film is then diced or otherwise discretized into a plurality of carriers 34.

The method (2) also may comprise a method 36 comprising the steps of preparing embossed mold 38, and cutting a film from a roll 40, followed by embossing the film 42. The embossed film is cut or diced or otherwise discretized to generate embossed pieces 44, which can then be sieved to a narrow size distribution 46. The carriers are then washed with a wash fluid such as water or a mixture of water and isopropyl alcohol to remove fine particles, followed by drying 48. The carriers are then subjected to a plasma treatment 50 in bulk accompanied by mixing to ensure uniformity of surface treatment 52 to form plasma treated embossed carriers. The methods (1) and (2) (as described above 22 and 36) can be modified to produce carriers on large scale using roll-to-roll operations for some or all of the steps of manufacturing. For example, the embossing or structure generation step can be scaled-up into a roll-to-roll operation, and the plasma treatment operation can be done in bulk in drum-style treaters, and the discretization can be done via roll-to-roll or sheet-fed die cutting operations.

Another example of a method for making the carriers comprises initially providing two flat polymer films. The method further comprises forming one or more structured indentations on the two flat polymer films individually on at least one surface of each of the two films, such as by embossing to make two embossed polymer films (embossed on one side each), and laminating the two embossed polymer films together, back to back, to form a composite laminated embossed polymer film, so that the outwardly facing surfaces comprise one or more of the structured indentations. The laminated embossed polymer film may then be diced to form a plurality of untreated carriers. The untreated carriers are then treated with a plasma treatment to form a plurality of plasma treated carriers. To create structured indentations, the flat polymer films may be alternatively be subjected to casting thermoforming, or injection molding, or a bulk polymer may be made into a solution and cast on a mold to form a film with the structured indentations.

The structured indentations may be formed in the carrier by one or more of the following methods. In one example, a textured roll is used to make the structured indentations on a heated polymer film in a roll-to-roll process. In another example, a flat mold is prepared by cutting or machining the negative of the desired indentations into a metal block. The metal block then may be used as-is or replicated first as a positive and then as a negative, using, for example, a polymer casting process. The negative mold can then be used in a batch-stamping or hot embossing process to emboss the pattern into a polymer film. In another example, a mold thus formed can be used in a solvent-casting process to make the polymer film with the structured indentations. A polymer solution can be coated on to the mold or textured roll, and dried and / or cured. The dried / cured film then peeled off to yield a film with the desired structured indentations. Alternate methods such as thermoforming or injection molding may also be used.

A cell culture system of the invention uses one or more of the carriers for growing cells. In one embodiment, the cell culture system is a bioreactor, more specifically, an agitated bioreactor. As mentioned herein, a bioreactor may refer to any device or system that supports cell growth. In one aspect, a bioreactor may refer to a device or a system for growing cells or tissues in the context of cell culture or tissue engineering. The bioreactor may employ agitation, generated by an internal impeller or paddle, or via externally rocking, rolling or shaking the culture vessel, or via bellows-induced motion of fluid. The bioreactor may, for example, be a reactor with rocking or rolling motion, such as Wave Bioreactor^{™}, a stirred tank bioreactor, a fluidized bed bioreactor, fixed bed bioreactor, a roller bottle or airlift bioreactor.

The Wave Bioreactor^{™} comprises a rocking platform supporting a vessel containing a culture fluid, wherein the culture fluid comprises cells in a culture media. The rocking motion of the platform induces mixing and mass transport in the culture fluid. A stirred tank bioreactor generally comprises an impeller system and optionally a sparging system to mix and aerate the culture. An airlift reactor relies on rising gas bubbles to mix and aerate the culture medium. Hydrodynamic factors such as mass transfer, mixing efficiency, and shear stress experienced by cells can be different in the different types of bioreactors. In addition, the cell growth rate and quality of cells may be influenced by operational differences between reactor types.

An example of a method of culturing adherent cells comprises providing one or more carriers for growing cells in a bioreactor, adding culture medium, adding an inoculum of cells to the carriers, allowing attachment of cells to the carriers, suspending the carriers in the medium continuously or intermittently, and allowing the cells to grow on the carriers. Cells may be grown in a culture flask prior to addition to the carriers. Cells may be grown on the carriers after extraction, for example, from blood, bone marrow or tissue section. In some other embodiments, the carriers may be introduced into a spinner flask, a stacked culture flask, a stirred tank reactor, a Wave bioreactor^{™} or any other in-vitro cell culture system.

Cultured cells may be detached or released from the carriers by a variety of methods. The cells may be released, for example, by using a mechanical method, an enzyme, a thermoresponsive polymer, a pH responsive polymer or a combination thereof. The cell release by mechanical method includes cell scraping. The cells may also be released by treating with proteolytic enzymes, such as trypsin. One non-enzymatic method uses calcium chelators, such as EDTA. Other non-enzymatic methods include, but are not limited to, physical methods that use ultrasound, which generates bubbles that facilitate cell detachment. Cultured cells from carriers comprising thermoresponsive polymers, such as poly-N-isopropylacrylamide (PNIPAAm) may be released by cooling the carrier to a temperature below LCST.

The carriers of the invention may be used for growing various adherent cells such as primary cells, stem cells and cell lines. In some embodiments, the adherent cells are shear-sensitive cells such as hMSCs. The cells may be derived from human tissue, for example, from adipose tissue, bone marrow or cord blood. Culture and release of multipotent and pluripotent cells with high purity, high efficiency and high yield are a current research and clinical need.

The carriers can be used in combination with a bioreactor or culture vessel, to provide or enhance surface area for the attachment and growth of anchorage-dependent cells. Some embodiments of the kit of the invention for culturing cells comprise a disposable housing or vessel pre-loaded with one or more carriers. In one embodiment, the carriers and the disposable housing or vessel may be provided separately. In one embodiment, the housing may be reusable. The housing may be, for example, a bag, a flask, a tank, a tube, a petridish or a bottle. The kit may further comprise culture media suitable for cell growth. The kit may comprise cells in a frozen condition and may further comprise a protocol for using the carriers.

### EXAMPLE 1. Fabrication of carrier for growing cells

*Method of making a pattern master*-A pattern-master was prepared by cutting grooves in a flat aluminum block using a dicing saw, which is outfitted with a resin-bonded diamond blade. A set of parallel grooves (the term being interchangeably used with 'indentations') was first cut in one direction, then a second set of parallel grooves was cut perpendicular to the first set of grooves. Finally, an effort was made to remove burrs that had formed in the first set of grooves during the cutting process. After the grooves were completed, the aluminum block was cleaned to remove any burrs on its surface. The pattern master determined the pattern geometry of the embossed carriers.

*Formation of first generation mold from the pattern master*-A first-generation mold was then made from the pattern-master using a fluorosilicone rubber, FSL 7661 (purchased from Momentive Performance Materials, Waterford, NY). To produce the first-generation mold, the two part fluorosilicone compound was mixed at a 1:1 ratio according to directions from the manufacturer, using a Hauschild SpeedMixer. The pattern-master was placed in a hollowed-out Teflon block and uncured fluorosilicone was applied, in excess, on the surface of the pattern master. A chrome-plated steel plate was placed on top of the fluorosilicone, and the fluorosilicone was cured in a heated hydraulic press at 4000 lb force and 170°C for 30 minutes. After cooling to room temperature, the cured fluorosilicone rubber-based first-generation mold was removed from the pattern-master and cured overnight at 200°C in air.

*Formation of second generation mold from the pattern master*- Two second-generation molds were then prepared using a silicone rubber-molding compound, RTV 664 (purchased from Momentive Performance Materials, Waterford, NY) from the first-generation mold as mentioned above. The silicone compound was mixed at a 10:1 ratio according to directions from the manufacturer, using a Hauschild SpeedMixer. The first-generation mold was placed inside a steel frame with the patterned surface up and the silicone compound was dispensed, in excess, on the first-generation mold. A flat stainless steel plate was placed on top of the silicone and the silicone was cured in a heated hydraulic press at 1000 lb force and 120°C for 30 minutes. After cooling to room temperature, the cured silicone rubber second-generation mold was removed from the fluorosilicone first-generation mold.

*Method of making embossed polystyrene sheets-* Multiple sheets of biaxially oriented polystyrene film (Trycite 1003U, Dow Chemical Company) were placed in between two second-generation molds with patterns facing in. The number of sheets of film was chosen so that the volume of polystyrene was sufficient to fill the pattern in the second-generation molds and still leave a small amount of polystyrene separating the molds. The films were then embossed (28, FIG. 3) in a heated hydraulic press with 1000 lb force and a temperature cycle that ramped up to 150°C for 5 minutes and then cooled to below 60°C. The embossing process fused the multiple sheets of film into a single monolithic structure that replicated the texture of the molds and pattern-master on both sides. The embossed polystyrene film was removed from the molds after cooling to room temperature.

*Chemical treatment of the embossed film surface-* To make the embossed polystyrene film compatible with cell growth, the film was O₂ plasma treated (30, FIG. 3) using a Plasma Therm SLR vacuum plasma reactor as mentioned in FIG. 3. Plasma treatment was performed on each side of the embossed film for 1 minute at 100 mtorr pressure using 100 sccm (Standard Cubic Centimeters per Minute) O₂ flow and 100 W forward radio frequency (RF) power in reactive ion etching (RIE) mode.

*Dicing of the film to generate carrier-* Carriers for cell culture were prepared from the plasma-treated embossed sheets either by manually cutting the film into 5mm x 5 mm pieces or 2 mm x 2 mm pieces, or by discretizing (44) and then sieving (46) to select a particular size range, or by punching circular discs of the desired size.

*Variants of the carrier fabrication process-* In some instances, a ceramic block was used in place of the aluminum block to make the pattern-master. A pattern-master was prepared by cutting grooves in a flat alumina block (99.6% alumina, fired, 20-25 µm polish from Acumet) using a dicing saw outfitted with a resin-bonded diamond blade. A set of parallel grooves was first cut in one direction, and then a second set of parallel grooves was cut perpendicular to the first set of grooves. The geometry of the pattern master determined the pattern geometry of the eventual embossed carriers. When the ceramic block was used, the first-generation mold was prepared slightly differently. Instead of the Teflon block, a steel frame was used to hold the ceramic pattern-master. The curing was performed at a higher temperature, 170°C for 15 minutes and then 200°C for 15 minutes. The rest of the procedure remained the same as described above.

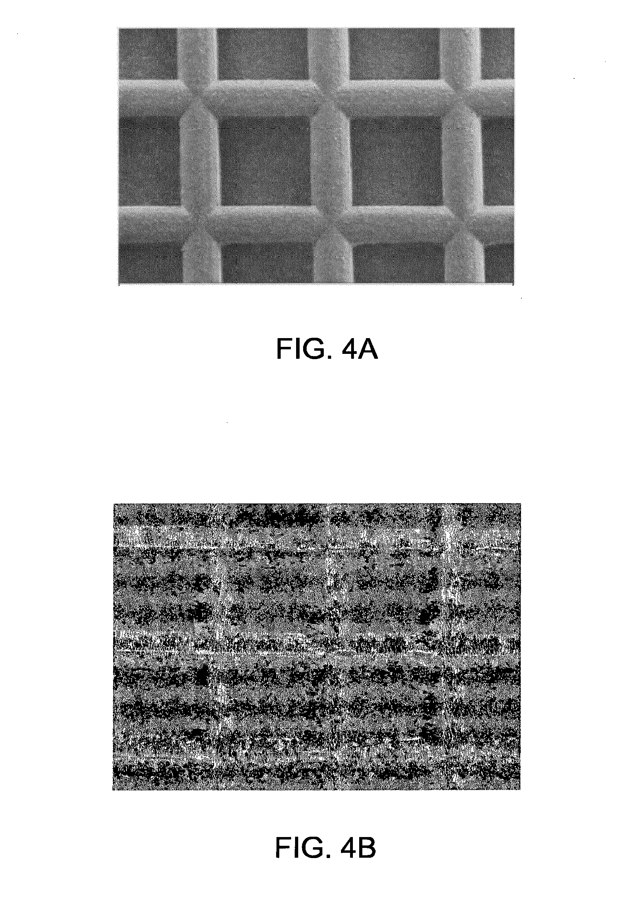

In some examples, the fluorosilicone first-generation molds were replaced with RTV silicone first-generation molds. The procedure was modified as described below. A first-generation mold was then made from the pattern-master using a silicone rubber-molding compound, RTV 664 from Momentive Performance Materials. To produce the first-generation mold, the silicone compound was mixed at a 10:1 ratio according to directions from the manufacturer, using a Hauschild SpeedMixer. The pattern-master was placed in a hollowed-out Teflon block and uncured silicone compound was applied, in excess, across the surface of the pattern master. A chrome-plated steel plate was placed on top of the silicone, and the silicone was cured in a heated hydraulic press at 1000 lb force and 120°C for 30 minutes. After cooling to room temperature, the cured silicone rubber first-generation mold was removed from the pattern-master. The first generation mold was coated with (tridecafluoro-1,1,2,2-tetrahydrooctyl) trichlorosilane by vacuum deposition at 750 mtorr for 45 minutes prior to making any second-generation molds. Cell carriers of different designs were made using the above fabrication procedures. The embossed cell carriers of the invention may include carriers with alternate wall shape. For example, carriers with rectangular shaped walls were made as shown in FIG. 4A, and carrier with triangular shaped wall in cross-section were made, as shown in FIG. 4B.

### EXAMPLE 2 Cell culture on the carrier and subsequent cell release

*Cell carriers*-The carriers used for the following examples had a length and width of 5mm, and a height of about 0.5mm. The carriers comprised a plurality of structured indentations on each of the two outer surfaces. Each of the structured indentations had a major axis and minor axis of 0.45mm each and a depth of 0.2mm.

*Cell culture*-The carriers for cell culture were used to culture and release CHO (Chinese Hamster Ovary, ATCC), MDCK (Madin-Darby Canine Kidney Cells, ATCC), MRC-5 (human lung fibroblast, ATCC), and hMSCs (human mesenchymal stem cells) cells. These cells were routinely cultured on polystyrene surfaces using the following media: F-12K (EMEM, Invitrogen) and 10% FBS (fetal bovine serum); and Eagle's minimum essential medium (EMEM, Invitrogen) and 10% FBS supplemented with 100U/mL penicillin-streptomycin (P/S, Invitrogen). Culture methods were performed at 37°C, in a humidified atmosphere of 5% CO₂. Cells were passaged by performing the steps of briefly rinsing the cell layer with PBS (phosphate buffered saline) followed by addition of 3.0 ml of 0.25% (w/v) Trypsin and 0.53 mM EDTA solution to the culture flask and observing the cells in an inverted microscope until the cell layer is dispersed. Subsequently, 7 ml of complete growth medium was added to the cells and the media, and the cells were mixed by gently pipetting several times. Appropriate aliquots of the cell suspension were transferred to new culture vessels with fresh media.

Cells used in the following experiments were freshly pre-cultured and harvested from cell culture flasks after growing in incubators at 37°C in a humidified, 5% CO₂ atmosphere. For static cell culture testing, pre-cultured cells were seeded at 2000 cells/cm² in 24-well plates with 1mL growth medium per well. Tissue culture treated plates (TCPS surface, Nunc) and or non-adherent plates (from Corning®) were used as control, wherein in the non-adherent plates (Corning®), disc-shaped embossed polystyrene carriers of the invention were inserted so as to fit snugly into the well. For cells grown under dynamic conditions on the carriers in stirred tank reactors (STR), pre-cultured cells were also seeded at 2000 cells/cm² in the carriers in 125mL disposable spinner flasks (Corning®). Cells and carriers were agitated at 60 rpm on spinner bases connected to timers to regulate the agitation cycle. Cells were subjected to agitation continuously or intermittently. In intermittent conditions, for example, the agitator was turned on for 1 min, and off for 45 min per cycle.

*Cell release by trypsin-* Cells were washed with PBS and harvested by trypsin-EDTA (Invitrogen, ∼10 minutes), when the cells were about 80-90% confluent. The trypsin was neutralized by addition of at least one volume of culture medium containing 10% serum, after the cells were released from the growth surface. After harvesting of the cells, cell number and cell viability were measured using a NucleoCounter® automated cell counter (from ChemoMetec).

### Qualitative and Quantitative estimation of cell growth

*Cell staining and imaging-* Samples for imaging were fixed at room temperature in 4% paraformaldehyde (PFA), which is freshly diluted in PBS from a 16% stock, stored in presence of argon in an amber glass vial. Once fixed, samples were stored at 4°C until they were stained and imaged. Fixed cells were stained with Hoechst 33342 dye (from Invitrogen) to highlight the nuclei and with phalloidin-Alexa-568 (from Invitrogen) to visualize the cytoskeleton (actin) after permeabilization with 0.1% Triton X-100 detergent (Sigma). The stained cells were imaged with an Nikon Eclipse TE2000-U inverted fluorescence microscope, wherein the microscope was fitted with appropriate filter cubes and light source for the fluorophores being used.

*Cell viability measurement by CellTiter-Glo®-* Cell growth and morphology was assessed at intervals by taking samples of carriers and either measuring total ATP content or fixing and staining for fluorescence microscopy. Cell growth was assayed by CellTiter-Glo® luminescent cell viability assay reagent from Promega, which determines the number of viable cells in culture based on quantitation of the ATP present, which signals the presence of metabolically active cells. The process involves adding a single reagent (CellTiter-Glo®) directly to cells cultured in serum-supplemented medium. The homogeneous reagent results in cell lysis and generation of luminescent signal proportional to the amount of ATP present. The amount of ATP is directly proportional to the number of cells present in the culture. The assay relies on thermostable luciferase, which generates a stable 'glow type' luminescent signal resulting from oxyluciferin catalysed by luciferase in presence of Mg⁺², ATP, and molecular oxygen. After 10 minutes of the cell lysis, 200µL aliquots of cell lysate were transferred to an opaque 96-well plate, mixed gently and read in a SpectraMax® luminescence microplate reader from Molecular devices to generate readings for cell viability. Luminescence readings from this assay are proportional to the number of viable cells present in the sample and so can be used to monitor the progress of cell growth.

### EXAMPLE 3-Characterization of Human mesenchymal stromal cell (hMSC) growth

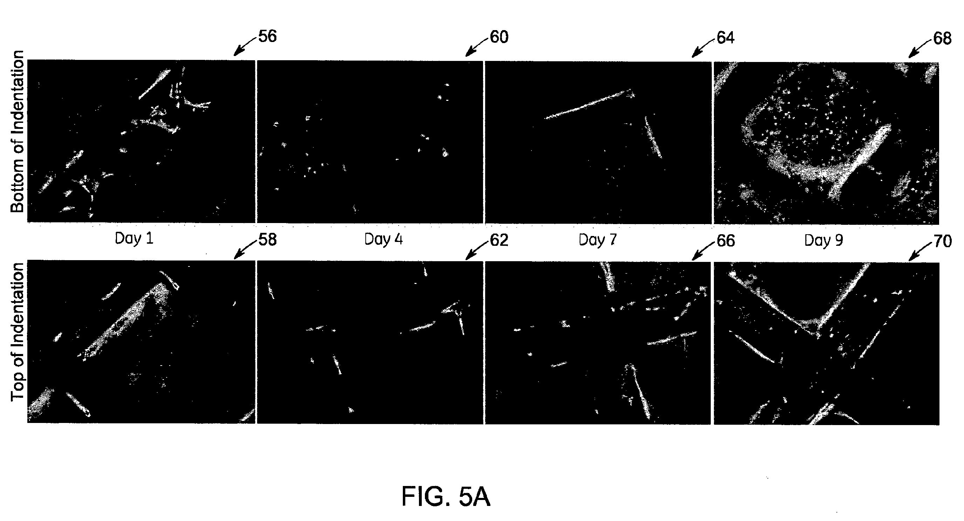

*hMSCs*- The hMSCs used for this experiment were purchased from Lonza (Part number PT-2501) (Basel, Switzerland). The hMSCs were grown on the carrier (interchangeably used herein as 'embossed carrier') in stirred tank reactors (STR). FIG. 5A shows the growth of cells on day 1 (56, 58), day 4 (60, 62), day 7 (64, 66), and day 9 (68, 70), clearly indicating an increase in cell count over time in culture. Cells were observed to grow on both the top and the bottom of the indentations of the embossed carrier. FIG. 5A further illustrates higher cell growth in the bottoms of the indentations than the tops. Cells were grown on embossed cell carriers in two different types of spinner flasks, one from Corning, and one from Wheaton (Magna-Flex®). Cells were grown on tissue culture polystyrene (TCPS) in static medium as a positive control.

### EXAMPLE 4- Quantitative estimation of hMSC growth

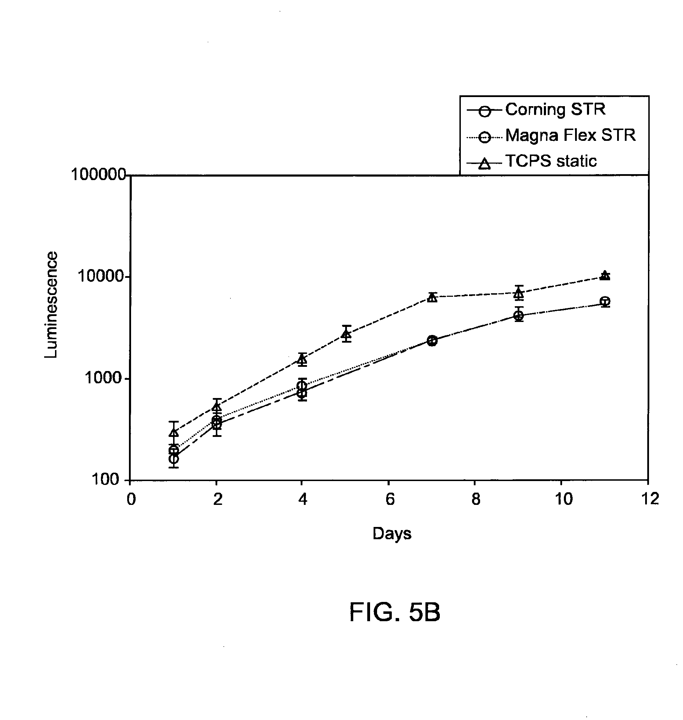

The cell growth was monitored via CellTiter-Glo® measurements and qualitatively via imaging. The growth rate of hMSCs on carriers in STR is comparable with that on TCPS, as shown in FIG. 5B. Luminescence of the cells represents cell number, and the luminescence increases with increasing cell count over time (FIG. 5B). Therefore, robust growth of hMSC on the carriers of the invention is demonstrated in FIG 5B for 11 days with a doubling time of ∼50 hours.

### EXAMPLE 5- Characterization of hMSC growth from various sources

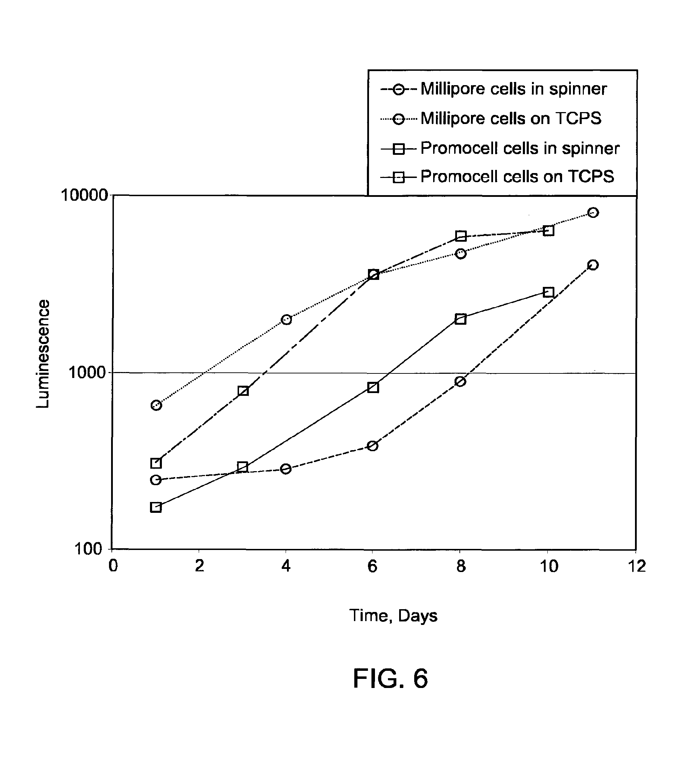

To demonstrate that the carriers support the growth of hMSCs from a variety of sources / donors, hMSCs were procured from 2 additional companies - (Poietics® Lonza cells have been referenced in examples 3 and 4 Promocell (part number C-12974) and Millipore (part number SCR108). FIG. 6 demonstrates robust hMSC cell growth, wherein the cells were purchased from Promocell and Millipore, and cultured in STR, and the growth is comparable to the cells grown on TCPS under static conditions.

### EXAMPLE 6- Characterization of hMSC growth in larger scale culture

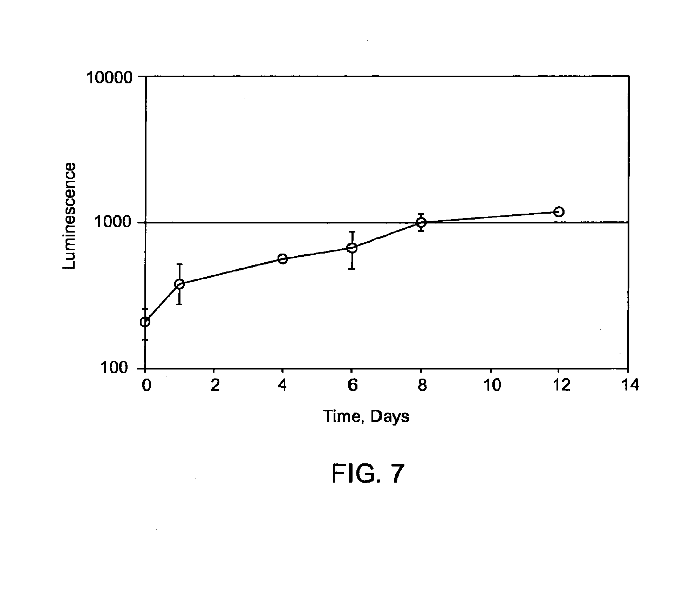

The hMSC culture was scaled up in 1L spinner flask with 500 ml of media. FIG. 7 shows robust hMSC (Lonza, part number PT-2501) growth for 12 days with a doubling time of about 85 hours.

### EXAMPLE 8- Characterization of Madin-Darby Canine Kidney (MDCK) Cell growth

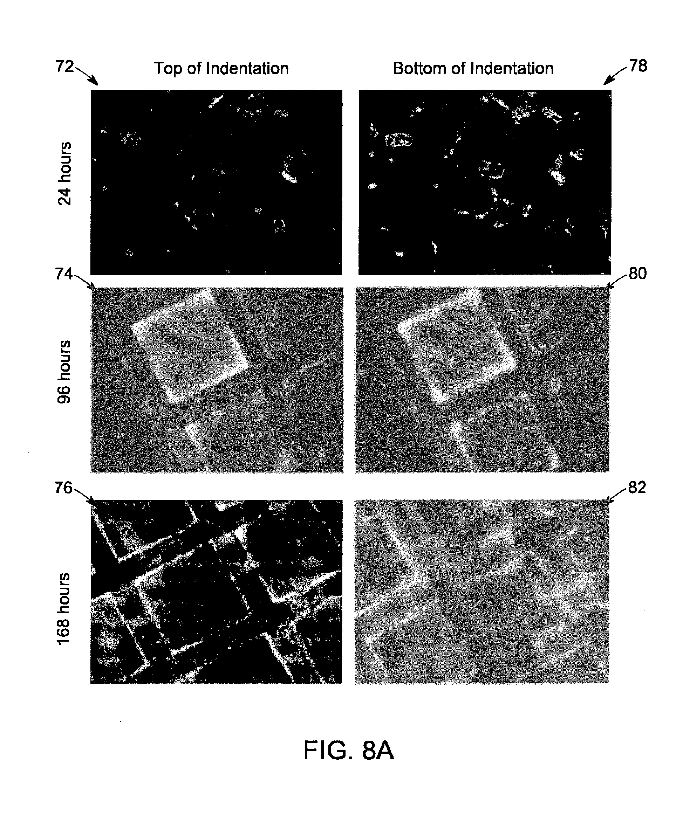

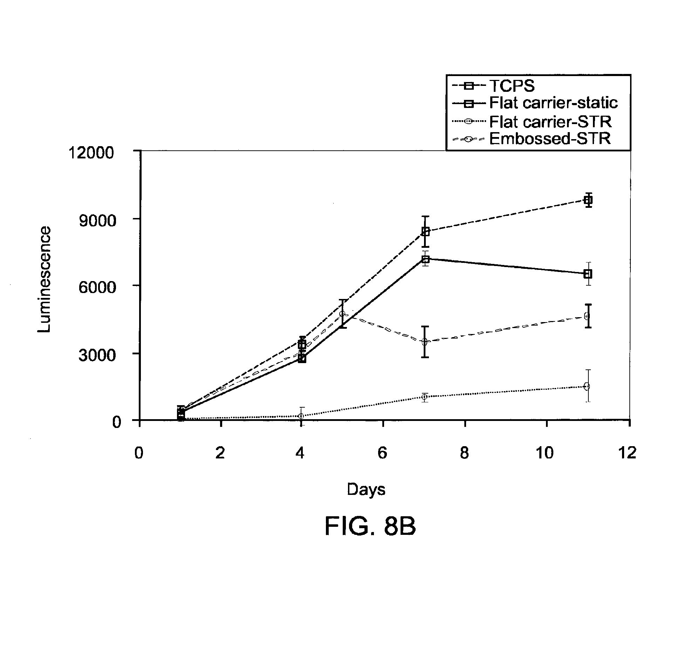

*Madin-Darby Canine Kidney Cells (MDCK)-* MDCK cell growth on carrier comprising plurality of indentations (or 'embossed carrier') is shown in FIGs. 8A and 8B. FIG. 8A shows MDCK cells growing at the top (72, 74, 76) and on the bottom (78, 80, 82) of the indentations in the carriers. Cell counts increases with time from 24 hours to 168 hours. Quantitative measurement of MDCK growth is presented in FIG.8B via CellTiter Glo® luminescence assay. As a comparative example, MDCK cells were grown on flat polystyrene carriers (with no indentations) in an STR with intermittent stirring. As controls, cell counts in static well plate (TCPS) and on the flat carriers in static conditions are also shown in FIG. 8B. MDCK cell growth on embossed carriers (marked as 'Embossed - STR' in FIG. 8B) was significantly higher than that on flat polystyrene carriers (marked as 'Flat carrier-STR' in FIG. 8B), as shown in FIG. 8B.

### EXAMPLE 9- Characterization of Human lung fibroblast cell growth

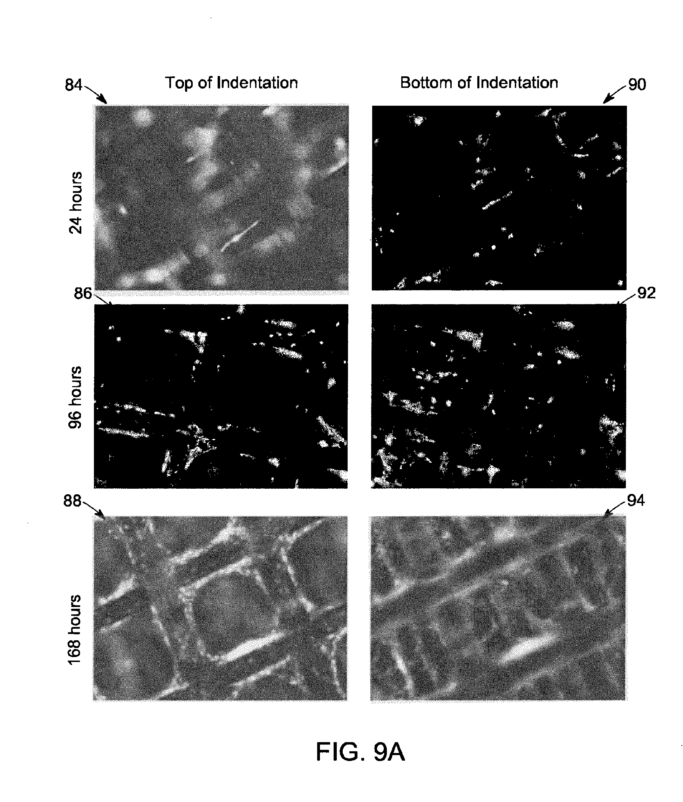

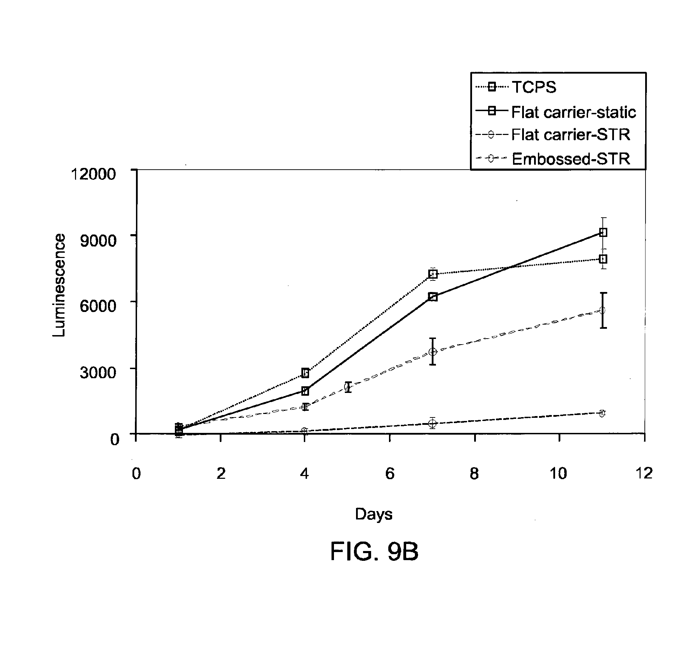

*Human lung fibroblast cells (MRC-5)-* Similar experiments were performed with MRC-5 cells (FIG. 9A) as mentioned above. In FIG. 9A, the qualitative measurement of MRC-5 cell growth at the top (84, 86, 88) and the bottom (90, 92, 94) of the indentations of the carriers is presented. Cell counts increase with time, from 24 (84 and 90) hours to 168 hours (88 and 94). Quantitative measurement of cell growth is presented in FIG.9B as CellTiterGlo® Luminescence (as described in above example). As a comparative example, MRC-5 cells were grown on flat polystyrene carriers (with no indentations) in an STR with intermittent stirring. As controls, cell counts in static well plate (TCPS) and on the flat carriers in static conditions are also shown in FIG. 9B. Cell growth on embossed cell carriers of the invention is greater than that on flat polystyrene cell carriers, as shown in FIG. 9B.

### EXAMPLE 10- Characterization of Chinese Hamster Ovary (CHO) cell growth

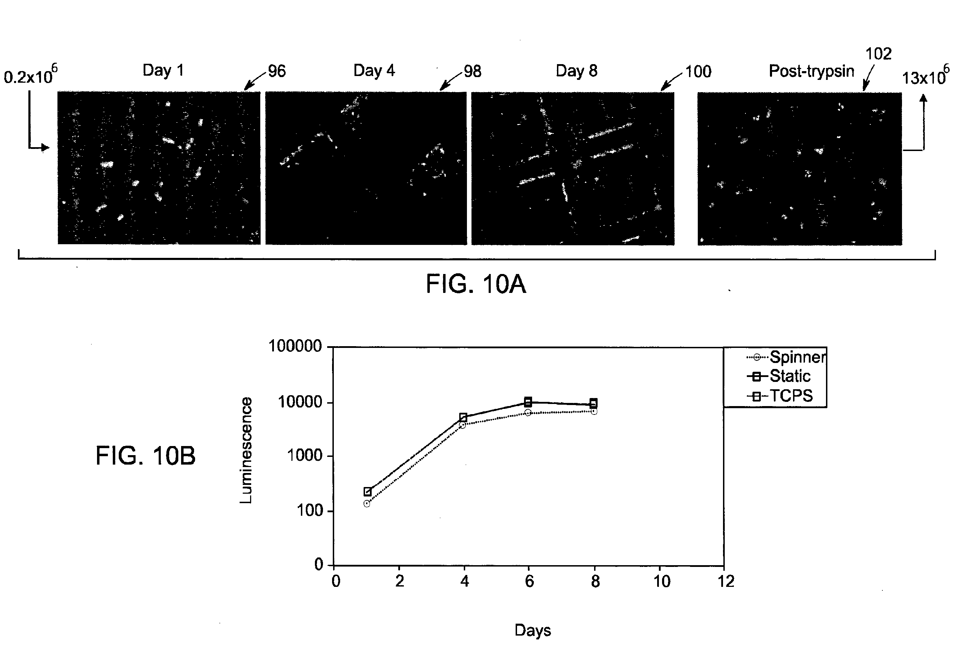

*Chinese Hamster Ovary (CHO)-* Similar cell growth experiments were performed using CHO cells on the carrier of the invention (FIG. 10A and 10B). Cells were imaged after 1 day (96), 4 days (98), 8 days (100), and post-trypsin treatment (102) as shown in FIG.10A. 0.2 X 10⁶ cells were seeded on day 0 (zero). Cell counts increased with time, and cell recovery after trypsin treatment was about 13 X 10⁶ as shown in FIG. 10A. The efficient release of CHO cells from the embossed carrier by standard method of trypsinization is also shown in FIG. 10A. The embossed carrier of the invention showed better cell release, compared to other commercially available carriers (data not shown). CHO cell growth on embossed cell carriers in a spinner flask, in a static well plate, and also on TCPS in static well plates is shown quantitatively in FIG. 10B. The cell growth in these three different conditions were comparable to each other (FIG. 10B), and CHO cells showed robust cell growth for 8 days with a doubling time of about 21 hours, with about 67 fold expansion as shown in FIG. 10B.

### Phenotypic and Functional Assays

In order to demonstrate that hMSCs grown on the carriers of the invention maintain their phenotype after expansion in a bioreactor, phenotypic characterization was done via adipogenic and osteogenic differentiation assays. Surface marker profile was also characterized via flow cytomtery.

### EXAMPLE 11- Adipogenic Differentiation Assays

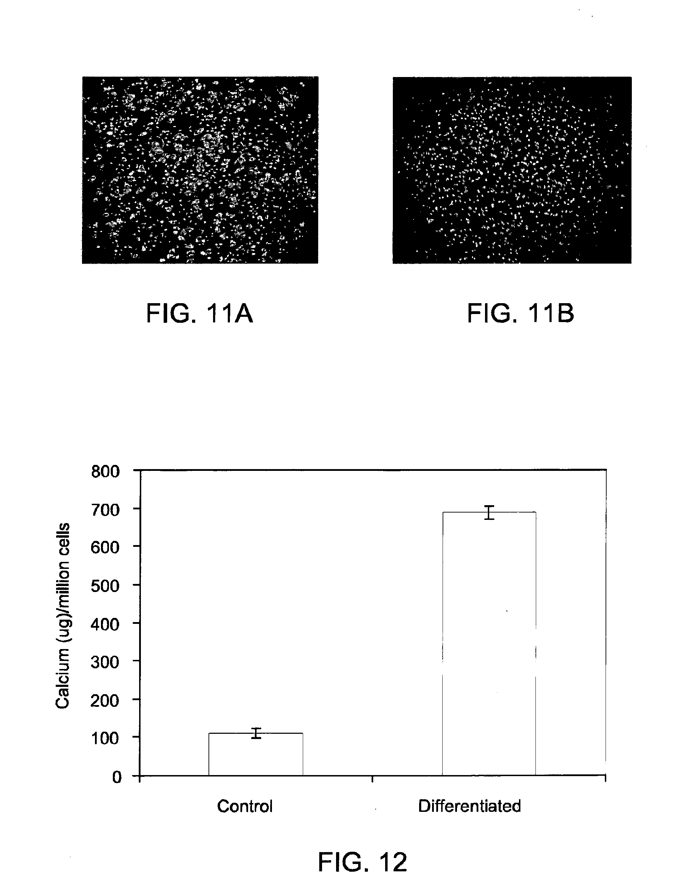

Adipogenesis-In order to demonstrate adipogenesis, MSCs were seeded at 2000-3000/well in a 96-well plate with growth medium same as used above to grow cells, Lonza MSCGM (part number PT-3001), with 3-6 wells per sample, and were grown until the cells became 100% confluent. The growth medium was replaced with adipogenic induction medium (Lonza, part number PT-3004) and incubated at 37°C for 3-4 days. After that, the adipogenic induction medium was replaced with maintenance or growth medium (Lonza) and incubated at 37°C for 3-4 days. These two steps were repeated two more times. Maintenance medium was added and replaced the medium on Day 3, and incubated for 7 days. The resulting fat cells were stained with Nile Red. The MSCs were maintained in maintenance medium as a negative control. The medium was removed and 4% paraformaldehyde (PFA) was added to fix cells for 5 minutes. After 5 minutes, the PFA was removed and Nile Red and Hoechst staining buffer were added, followed by incubation for 30-60mins. Buffers were prepared using Nile Red stock at the ratio of 1:1000 in DMSO (1mg/ml in DMSO), Hoechst buffer solution at the ratio of 1:1000, and 0.1% of Triton X100 in phosphate buffered saline (PBS). The cells were rinsed with PBS twice by adding 0.2 ml PBS per well. The cells were imaged with Nikon Eclipse TE2000-U inverted fluorescence microscope using DAPI and Cy3 stains. FIG. 11A shows red vacuoles indicating accumulation of lipid, with respect to the control MSCs as shown in FIG. 11B. The accumulation of lipid in the vacuoles indicates that cells grown on these carriers in bioreactor conditions retain the capability to differentiate into adipocytes which is a property expected of MSCs. hMSCs from Lonza were used in this experiment. Cells were grown as described in example 3.

### EXAMPLE 12- Osteogenic Differentiation Assays

Osteogenesis-The MSCs were seeded in 24-well tissue culture plate at a concentration of 20-30k/well at three sets, and cells were cultured in growth medium at 37°C overnight. The growth medium was replaced with osteogenesis induction medium and the cells were fed with the same medium after every 3 days. This procedure was continued for 2-3 weeks. The morphology change from spindle to cubical shape was noted. The calcium content was measured by following method. The cells were washed in Ca2+-free PBS, and 100µl of 0.5M HCl added to dissolve the cells homogenously, and 70µl aliquots were added to a 96-well plate. 100µl Color Reagent was added followed by 100µl Base Reagent (STANBIO, Cat#0150-250, Calcium Liquid Color). The reagents were mixed and incubated for 1min. The optical density (O.D) was measured at 550nm within 60 minutes after mixing the reagents. FIG. 12 shows the calcium content of differentiated osteogenic cells is about 7 times higher than that of the control cells. The increased calcium content in the differentiated cells indicates that the MSCs grown on these carriers retain the ability to differentiate into an osteoblastic phenotype which is expected of cells defined as MSCs.

### EXAMPLE 13 Characterization of MSCs by Flow Cytometry analysis (FACS)

hMSCs are expected to express a number of surface protein markers and simultaneously lack a number of others. Positive markers include CD105, CD90, CD166, CD29, CD44 and CD73 while negative markers include CD14, CD29, CD19, CD45 and CD3. For each marker, the assay contained at least 150,000 cells in test tube. The cells were blocked in PBS and 10% normal goat serum (NGS) for 30 min at 4°C. Antibodies were used for the following cell surface markers: CD105, CD90, CD166, CD45, CD73, CD34, CD44, CD14, CD29, CD19 and CD31. hMSCs from Millipore (part number SCR108) were grown in static T-flask (control) or a spinner flask on the embossed carriers using conditions as described in example 3. After harvesting with trypsin, the cells were incubated with labeled antibody for 1 hour at 4°C in PBS and 1% NGS and then washed with PBS and 1% NGS by centrifugation to pellet down the cells. The pellet was re-suspended in 150µL PBS and 1% NGS buffer. The cells were analyzed by flow cytometer according to manufacturer's instructions (Beckman Coulter- Cytomics FC500MPL). hMSCs and the manufacturer's growth medium were sourced from Millipore and cells were grown as described in example 5.

The results of the FACS analysis are shown in Table 1 below which indicates that MSCs grown on the embossed carriers in bioreactor conditions showed a marker profile consistent with what is expected of these cells (also shown by the control cells grown under static conditions in T-flasks). The top row of the table indicates the specific markers assayed by FACS. Plus (+) symbol indicates that marker was present (>90%) and the minus (-) sign indicates a marker that was not detected (<5%). The expression of the 6 positive markers listed above are designated positive if expression was observed in >90% of the cells while the 5 negative markers were designated as such if expression was observed for <5%of the cells.

**Table 1**

| | 105 | 166 | 29 | 44 | 90 | 73 | 14 | 19 | 31 | 34 | 45 |
|---|---|---|---|---|---|---|---|---|---|---|---|
| control | + | + | + | + | + | + | - | - | - | - | - |
| Spinner flask | + | + | + | + | + | + | - | - | - | - | - |

While only certain features of the invention have been illustrated and described herein, many modifications and changes will occur to those skilled in the art. It is, therefore, to be understood that the appended claims are intended to cover all such modifications and changes as fall within the scope of the invention.

## Claims

1. A carrier for growing adherent cells, comprising:
two outer surfaces; and
one or more structured indentations on the two outer surfaces, wherein the carrier has a length at least about 0.2 mm, a width at least about 0.2 mm, and a height in a range from about 0.05 mm to 1.2 mm and each of the structured indentations has a major axis in a range from about 0.1 mm to 0.5 mm, a minor axis in a range from about 0.1 mm to 0.5 mm and a depth in a range from about 0.025 mm to about 0.5 mm.

2. A cell culture system comprising one or more of the carriers of claim 1, wherein the cell culture system is a bioreactor.

3. A kit for culturing cells, comprising a disposable housing pre-loaded with the carrier of claim 1.

4. A carrier according to claim 1, comprising:
a single structured indentation on at least one surface, wherein the single structured indentation has a length of at least about 1 mm, a width of at least about 1mm, a height in a range from about 1mm to 10mm, and a wall-thickness of the carrier in a range from about 0.05 mm to 2 mm.

5. A method of culturing adherent cells, comprising:
providing a carrier for growing the cells, comprising two outer surfaces; and one or more structured indentations on the two outer surfaces, wherein the carrier has a length at least about 0.2 mm, a width at least about 0.2 mm, and a height in a range from about 0.05 mm to 1.2mm and each of the structured indentations has a major axis in a range from about 0.1 mm to 0.5 mm, minor axis in a range from about 0.1 mm to 0.5 mm and depth in a range from about 0.025 mm to about 0.5 mm, and
growing the cells on the carrier.

6. A method of making a carrier for growing cells, comprising:
a) providing two flat polymer films;
b) forming on the two flat polymer films, a plurality of structured indentations on at least one surface of each of the two films;
c) laminating the two polymer films together so that at least two outwardly facing surfaces comprise a plurality of the structured indentations;
d) cutting the laminated polymer film into a plurality of portions; and
e) imparting a treatment to the portions comprising one or more of corona discharge treatment, gas plasma treatment, chemical functionalization or coating.

7. The method of claim 6 wherein the carrier is cytophilic.

## Patentansprüche

1. Träger zum Kultivieren von anhaftenden Zellen, umfassend:
zwei Außenflächen; und
eine oder mehrere strukturierte Einschnitte auf den zwei Außenflächen, wobei der Träger eine Länge von mindestens etwa 0,2 mm, eine Breite von mindestens etwa 0,2 mm und eine Höhe in einem Bereich von etwa 0,05 mm bis 1,2 mm hat und jede der strukturierten Einschnitte eine Hauptachse in einem Bereich von etwa 0,1 mm bis 0,5 mm, eine Nebenachse in einem Bereich von etwa 0,1 mm bis 0,5 mm und eine Tiefe in einem Bereich von etwa 0,025 mm bis etwa 0,5 mm hat.

2. Zellkultursystem, das einen oder mehrere der Träger nach Anspruch 1 umfasst, wobei das Zellkultursystem ein Bioreaktor ist.

3. Set zum Kultivieren von Zellen, das ein wegwerfbares Gehäuse umfasst, welches mit dem Träger von Anspruch 1 vorgeladen wird.

4. Träger nach Anspruch 1, umfassend:
einen einfachen strukturierten Einschnitt auf mindestens einer Fläche, wobei der einfache strukturierte Einschnitt eine Länge von mindestens etwa 1 mm, eine Breite von mindestens etwa 1 mm, eine Höhe in einem Bereich von etwa 1 mm bis 10 mm und eine Wanddicke des Trägers in einem Bereich von etwa 0,05 mm bis etwa 2 mm hat.

5. Verfahren zum Kultivieren von anhaftenden Zellen, umfassend:
providing a carrier for growing the cells, comprising two outer surfaces; and one or more structured indentations on the two outer surfaces, wherein the carrier has a length at least about 0.2 mm, a width at least about 0.2 mm, and a height in a range from about 0.05 mm to 1.2mm and each of the structured indentations has a major axis in a range from about 0.1 mm to 0.5 mm, minor axis in a range from about 0.1 mm to 0.5 mm and depth in a range from about 0.025 mm to about 0.5 mm, and
growing the cells on the carrier.

6. A method of making a carrier for growing cells, comprising:
a) Vorsehen von zwei flachen Polymerfilmen;
b) auf den zwei flachen Polymerfilmen, Bilden mehrerer strukturierter Einschnitte auf mindestens einer Fläche von jedem der zwei Filme;
c) Verbinden der zwei Polymerfilme durch Laminieren, so dass mindestens zwei nach außen weisende Flächen mehrere der strukturierten Einschnitte umfassen;
d) Schneiden des laminierten Polymerfilms in mehrere Portionen; und
e) Vermitteln einer Behandlung für die Teile, die eine oder mehrere aus einer Koronaentladungsbehandlung, Gasplasmabehandlung, chemischen Funktionalisierung oder Beschichtung umfasst.

7. Verfahren nach Anspruch 6, wobei der Träger zytophil ist.

## Revendications

1. Support pour faire croître des cellules adhérentes, comprenant :
deux surfaces extérieures ; et
une ou plusieurs encoches structurées sur les deux surfaces extérieures, dans lequel le support a une longueur d'au moins environ 0,2 mm, une largeur d'au moins environ 0,2 mm, et une hauteur dans une plage d'environ 0,05 mm à 1,2 mm et chacune des encoches structurées a un axe principal dans une plage d'environ 0,1 mm à 0,5 mm, un axe secondaire dans une plage d'environ 0,1 mm à 0,5 minute et une profondeur dans une plage d'environ 0,025 mm à environ 0,5 mm.

2. Système de culture de cellules comprenant un ou plusieurs des supports de la revendication 1, dans lequel le système de culture de cellules est un bioréacteur.

3. Kit pour la culture de cellules, comprenant un logement jetable préchargé avec le support de la revendication 1.

4. Support selon la revendication 1, comprenant :
une encoche structurée unique sur au moins une surface, dans lequel l'encoche structurée unique a une longueur d'au moins environ 1 mm, une largeur d'au moins environ 1 mm, une hauteur dans une plage d'environ 1 mm à 10 mm, et une épaisseur de paroi du support dans une plage d'environ 0,05 mm à 2 mm.

5. Procédé de culture de cellules adhérentes, comprenant :
la fourniture d'un support pour cultiver les cellules, comprenant deux surfaces extérieures ; et une ou plusieurs encoches structurées sur les deux surfaces extérieures, dans lequel le support a une longueur d'au moins environ 0,2 mm, une largeur d'au moins environ 0,2 mm, et une hauteur dans une plage d'environ 0,05 mm à 1,2 mm et chacune des encoches structurées a un axe principal dans une plage d'environ 0,1 mm à 0,5 mm, un axe secondaire dans une plage d'environ 0,1 mm à 0,5 mm et une profondeur dans une plage d'environ 0,025 mm à environ 0,5 mm, et
croissance des cellules sur le support.

6. Procédé de fabrication d'un support pour faire croître des cellules, comprenant :
a) la fourniture de deux films de polymère plats ;
b) la formation sur les deux films de polymère plats d'une pluralité d'encoches structurées sur au moins une surface de chacun des deux films ;
c) le laminage des deux films de polymère ensemble de sorte qu'au moins deux surfaces faisant face vers l'extérieur comprennent une pluralité des encoches structurées ;
d) la découpe du film de polymère stratifié en une pluralité de parties ; et
e) l'application d'un traitement aux parties comprenant un ou plusieurs d'un traitement par décharge par effet corona, d'un traitement au gaz de plasma, d'un revêtement ou d'une fonctionnalisation chimique.

7. Procédé selon la revendication 6, dans lequel le support est cytophile.
